(19)

**Europäisches Patentamt**
**European Patent Office**
**Office européen des brevets**

(11) **EP 4 779 304 A1**

(12) **EUROPEAN PATENT APPLICATION**
published in accordance with Art. 153(4) EPC

(43) Date of publication:
**22.07.2026 Bulletin 2026/30**

(21) Application number: **25864009.3**

(22) Date of filing: **03.09.2025**

(51) International Patent Classification (IPC):
**G01N 33/44** (2006.01)     **C12Q 1/02** (2006.01)
**C12Q 1/34** (2006.01)

(52) Cooperative Patent Classification (CPC):
**C12Q 1/02; C12Q 1/34; G01N 33/44**

(86) International application number:
**PCT/KR2025/013565**

(87) International publication number:
**WO 2026/054507 (12.03.2026 Gazette 2026/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **03.09.2024 KR 20240119312
03.09.2024 KR 20240119313
03.09.2024 KR 20240119314
11.12.2024 KR 20240183657**

(71) Applicant: **LG Chem, Ltd.
Seoul 07336 (KR)**

(72) Inventors:
• **JEONG, Hee Jeong
Daejeon 34122 (KR)**
• **KIM, Dohyung
Daejeon 34122 (KR)**
• **LEE, Jeongbin
Daejeon 34122 (KR)**

• **BAK, Gwangbae
Daejeon 34122 (KR)**
• **PARK, Jeseob
Daejeon 34122 (KR)**
• **SHIN, Minhwa
Daejeon 34122 (KR)**
• **LIM, Young Hee
Daejeon 34122 (KR)**
• **KIM, Hyunjung
Daejeon 34122 (KR)**
• **BAEK, Song Yi
Daejeon 34122 (KR)**
• **JANG, Se Young
Daejeon 34122 (KR)**
• **JEONG, Byeong Joon
Daejeon 34122 (KR)**

(74) Representative: **Plasseraud IP
104 Rue de Richelieu
CS92104
75080 Paris Cedex 02 (FR)**

(54) **ENZYME SOLUTION AND METHOD FOR EVALUATING BIODEGRADABILITY OF BIODEGRADABLE RESIN USING SAME**

(57)     The present disclosure relates to an enzyme solution having biodegradable resin degradability and being useful for evaluating the biodegradability of biodegradable resin, and a biodegradability evaluation method for biodegradable resin using the same.

【FIG. 1】

EP 4 779 304 A1

**Description**

[Technical Field]

Cross-reference to Related Application(s)

This application claims the benefit of priority based on Korean Patent Application Nos. 10-2024-0119312, 10-2024-0119313, and 10-2024-0119314 filed on September 3, 2024, and Korean Patent Application No. 10-2024-0183657 filed on December 11, 2024, the entire contents of which disclosed in the documents of said Korean patent applications are incorporated as a part of the present specification.

The present disclosure relates to an enzyme solution having biodegradable resin degradability and being useful for evaluating the biodegradability of biodegradable resin, and a biodegradability evaluation method for biodegradable resin using the same.

[Background Art]

Conventional petroleum-based plastic materials do not decompose naturally and cause serious environmental pollution. Accordingly, biodegradable polymers capable of biodegradation in nature, such as polylactide (PLA), poly(butylene adipate-co-terephthalate) (PBAT), and polybutylene succinate (PBS), are attracting attention as substitutes for petroleum-based plastic materials.

In developing such biodegradable resin, biodegradability evaluation is essential. However, existing biodegradability tests are conducted over several weeks to several months depending on each condition such as soil, compost, or marine, and there is a problem that expensive large-scale equipment is required.

Therefore, there is a demand for the development of an evaluation method capable of evaluating the biodegradability of biodegradable resin in a short period of time using a simpler method.

[Detailed Description of the Invention]

[Technical Problem]

It is an object of the invention to provide an enzyme solution having degradability toward biodegradable resin and enabling simpler and faster prediction of biodegradability when biodegradable resin is exposed to a natural environment.

Also, it is an object of the invention to provide a biodegradability evaluation method for biodegradable resin using the enzyme solution.

[Technical Solution]

According to one embodiment of the present disclosure, there is provided an enzyme solution comprising: a hydrolase comprising lipase and cutinase; and phosphate-buffered saline.

According to another embodiment of the present disclosure, there is provided a biodegradability evaluation method for biodegradable resin comprising the steps of: i) contacting an enzyme solution comprising a hydrolase comprising lipase and cutinase, and phosphate-buffered saline with a biodegradable resin; and ii) observing a degradation state of the biodegradable resin over time.

[Advantageous Effects]

The enzyme solution of the present disclosure has biodegradable resin degradability and can be usefully used for evaluating the biodegradability of biodegradable resin. According to the biodegradability evaluation method for biodegradable resin of the present disclosure using the enzyme solution, the biodegradability of biodegradable resin can be evaluated simply and quickly without expensive large-scale equipment. In addition, according to the biodegradability evaluation method for biodegradable resin, the biodegradability of biodegradable resin under various natural environmental conditions such as soil, compost, and marine can be easily predicted.

[Brief Description of Drawings]

FIG. 1 shows the biodegradability evaluation results in a soil/compost environment of poly(butylene adipate-co-terephthalate) compound 1 (PBAT C1) and poly(butylene adipate-co-terephthalate) compound 2 (PBAT C2) performed in (3) of Example A-1.

FIG. 2 shows the biodegradability test results under industrial composting conditions of PBAT C1, PBAT C2, and cellulose (manufacturer: Sigma-Aldrich, product number: 310697) measured according to the ISO 14855-1 standard.

FIG. 3 shows the biodegradability evaluation results in a marine environment of poly(butylene succinate) (PBS) and poly(butylene succinate adipate) (PBSA) performed in (3) of Example A-2.

FIG. 4 shows the biodegradability test results under marine conditions of PBS and PBSA measured according to the ASTM D6691 standard.

FIG. 5 shows the biodegradability evaluation results in a soil/compost environment of the PBAT/TPS compound and PBAT/PLA compound performed in (3) of Example B-1.

FIG. 6 shows the biodegradability evaluation results in a soil environment according to the ISO 17556 standard for the PBAT/TPS compound, PBAT/PLA compound, and cellulose (manufacturer: Sigma-Aldrich, product number: 310697) used in Example B-1.

FIG. 7 shows the biodegradability evaluation results under marine conditions of PBS and PBSA performed in (3) of Example B-2.

FIG. 8 is a schematic diagram showing the method of dropping the enzyme solution onto a specimen in the biodegradability evaluation of Examples C-1 and C-2.

FIG. 9 shows the biodegradability evaluation results in an industrial compost environment according to the ISO 14855 standard for PBAT compound 1 (PBAT C1), PBAT compound 2 (PBAT C2), and cellulose (manufacturer: Sigma-Aldrich, product number: 310697) used in Example C-1.

FIG. 10 shows the biodegradability evaluation results in a soil/compost environment of PBAT compound 1 and PBAT compound 2 of (1) Example C-1, (2) Comparative Example C-1, and (3) Comparative Example C-2.

FIG. 11 shows the biodegradability evaluation results in a marine environment according to the ASTM D6691 standard for P3HP 1, P3HP 2, and cellulose (manufacturer: Sigma-Aldrich, product number: 310697) used in Example C-2.

FIG. 12 shows the biodegradability evaluation results in a marine environment of P3HP 1 and P3HP 2 performed in (4) of Example C-2.

FIG. 13 is an image showing the degree of biodegradation of biodegradable resin obtained according to an embodiment of the present disclosure.

FIG. 14 is an image showing the degree of biodegradation of biodegradable resin obtained according to an embodiment of the present disclosure.

FIG. 15 is a graph showing the difference in image brightness over time obtained according to an embodiment of the present disclosure.

FIG. 16 is a graph showing a first derivative function and a second derivative function obtained according to an embodiment of the present disclosure.

FIG. 17 shows the biodegradability evaluation results in home compost for PBSA compound 1 (PBSA C1), PBSA compound 2 (PBSA C2), PBSA compound 3 (PBSA C3), PBSA compound 4 (PBSA C4), and cellulose (manufacturer: Sigma-Aldrich, product number: 310697) measured in accordance with the ISO 14855-1 standard.

[Detailed Description of the Embodiments]

Terms used in the present specification are used only to describe exemplary embodiments and are not intended to limit the invention. Singular expressions include plural expressions unless the context clearly indicates otherwise. In the present specification, terms such as "comprise", "provide", or "have" are intended to specify the presence of implemented features, steps, components, or combinations thereof, and it should be understood that they do not preclude in advance the possibility of existence or addition of one or more other features, steps, components, or combinations thereof.

Since the invention can be modified in various ways and can have various forms, specific embodiments will be exemplified and described in detail below. However, this is not intended to limit the invention to a specific disclosed form, and it should be understood to include all changes, equivalents, or substitutes included in the spirit and technical scope of the invention.

In the present disclosure, "biodegradable resin" or "biodegradable polymer" means a polymer that is decomposed by microorganisms into natural by-products such as water, carbon dioxide, nitrogen, biomass, and inorganic salts.

Hereinafter, the present disclosure will be described in detail.

## Enzyme solution

According to an embodiment of the present disclosure, there is provided an enzyme solution comprising: a hydrolase comprising lipase and cutinase; and phosphate-buffered saline.

The enzyme solution of the present disclosure has biodegradable resin degradability and can be usefully used to

EP 4 779 304 A1

evaluate the biodegradability of biodegradable resin in a short period of time.

In particular, by adjusting the type and/or content of the hydrolase included in the enzyme solution, various environments such as a soil environment, a compost environment, and a marine environment can be implemented, thereby predicting the biodegradability of biodegradable resin under each environmental condition.

As a solvent for preparing the enzyme solution according to an embodiment of the present disclosure, phosphate-buffered saline (PBS) can be used.

As an example, the phosphate-buffered saline (PBS) may contain 137 mM NaCl, 2.7 mM KCl, 8 mM $Na_2HPO_4$, and 2 mM $KH_2PO_4$ as a mixture of ultrapure grade phosphate buffer adjusted to pH 7.4 and saline.

In one embodiment, the lipase may include lipase derived from one or more microorganisms selected from the group consisting of *Pseudomonas Cepacia, Rhizopus Oryzae, Aspergillus niger,* and *Aspergillus Oryzae.*

In one embodiment, the lipase may include lipase of *Pseudomonas Cepacia* and/or lipase of *Rhizopus Oryzae.*

Also, the cutinase may include cutinase derived from one or more microorganisms selected from the group consisting of *Humicola Insolens, Aspergillus Oryzae, Fusarium solani,* and *Pseudomonas putida.*

In one embodiment, the cutinase may include cutinase of *Humicola Insolens* and/or cutinase of *Aspergillus Oryzae.*

In one embodiment, the lipase may include lipase of *Pseudomonas Cepacia,* and the cutinase may include cutinase of *Humicola Insolens.*

When it is desired to evaluate the biodegradability of biodegradable resin in soil and compost (industrial or home compost) environments, it is preferable to include lipase of *Pseudomonas Cepacia* and lipase of *Rhizopus Oryzae* as the lipase. In addition to this, lipase of *Aspergillus niger* and/or lipase of *Aspergillus Oryzae* may be further included.

When it is desired to evaluate the biodegradability of biodegradable resin in soil and compost (industrial or home compost) environments, it is preferable to include cutinase of *Humicola Insolens* and cutinase of *Aspergillus Oryzae* as the cutinase. In addition to this, cutinase of *Fusarium solani* and/or cutinase of *Pseudomonas putida* may be further included.

When it is desired to evaluate the biodegradability of biodegradable resin in a marine environment, it is preferable to include lipase of *Pseudomonas Cepacia* as the lipase. In addition to this, lipase of *Aspergillus* niger and/or lipase of *Aspergillus Oryzae* may be further included.

When it is desired to evaluate the biodegradability of biodegradable resin in a marine environment, it is preferable to include cutinase of *Humicola Insolens* and cutinase *of Aspergillus Oryzae* as the cutinase. In addition to this, cutinase of *Fusarium solani* and/or cutinase of *Pseudomonas putida* may be further included.

Meanwhile, the enzyme solution may further include additional enzymes in addition to the lipase and cutinase. As such additional enzymes, at least one selected from the group consisting of $\alpha$-amylase, cellulase, proteinase K, protease, acetyl-CoA carboxylase (ACCase), and alcalase can be used.

Among the additional enzymes, $\alpha$-amylase can be applied to resins containing starch such as TPS and CMS, cellulase can be used for resins containing cellulose such as CMC, and proteinase K, protease, and alcalase can be applied to resins containing polylactic acid.

In one embodiment, as the $\alpha$-amylase, $\alpha$-amylase of *Bacillus Licheniformis* and/or $\alpha$-amylase of *Aspergillus oryzae* may be used; as the cellulase, cellulase of *Trichoderma reesei* and/or cellulase of *Aspergillus sp.* may be used; as the proteinase K, proteinase K of *Tritirachium album* may be used; as the protease, protease of *Bacillus licheniformis* may be used; and as the alcalase, alcalase of *Bacillus licheniformis* may be used. These enzymes can all be used when simulating soil and compost environments or marine environments.

In one embodiment, an enzyme solution for evaluating the biodegradability of biodegradable resin in soil and compost (industrial or home compost) environments includes lipase of *Pseudomonas Cepacia,* lipase of *Rhizopus Oryzae,* cutinase of *Humicola Insolens,* and cutinase of *Aspergillus Oryzae,* and may optionally further include $\alpha$-amylase of *Bacillus Licheniformis.*

In one embodiment, an enzyme solution for evaluating the biodegradability of biodegradable resin in a marine environment includes lipase of *Pseudomonas Cepacia* and cutinase of *Humicola Insolens,* and may optionally further include cutinase of *Aspergillus Oryzae.*

The amount of each enzyme included in the enzyme solution can be appropriately selected depending on the environmental conditions to be simulated and the biodegradable resin being evaluated.

However, the total amount of hydrolase per 1 ml of the enzyme solution is preferably 400 unit or more, or 800 unit or more, or 1,000 unit or more, or 2,000 unit or more, or 3,000 unit or more, or 4,000 unit or more, or 6,000 unit or more, or 8,000 unit or more, and 80,000 unit or less, or 60,000 unit or less, 40,000 unit or less, or 30,000 unit or less, or 20,000 unit. If the amount of hydrolase in the solution is too small, the decomposition of the biodegradable resin is slowed down, making rapid biodegradability evaluation difficult. Conversely, if the amount of hydrolase in the solution is excessively large, the biodegradable resin decomposes too quickly, making it difficult to determine the relative difference, which is not suitable for biodegradability evaluation.

Specifically, when it is desired to evaluate the biodegradability of biodegradable resin in soil and compost environments, the cutinase may be 10 unit or more, or 20 unit or more, or 100 unit or more, or 200 unit or more, or 500 unit or more, or 1,000 unit or more, or 2,000 unit or more, and 40,000 unit or less, or 30,000 unit or less, 20,000 unit or less, or 15,000 unit or less, or 10,000 unit or less, or 8,000 unit or less, or 4,000 unit or less, and the lipase may be 10 unit or more, or 20 unit or more, or 800 unit or more, or 1,500 unit or more, or 1,600 unit or more, or 3,000 unit or more, and 40,000 unit or less, or 30,000 unit or less, or 20,000 unit or less, or 15,000 unit or less, or 10,000 unit or less, or 8,000 unit or less, or 4,000 unit or less.

In the enzyme solution, the enzyme unit ratio of lipase:cutinase may be 0.5:1 or more, 0.8:1 or more, or 1:1 or more, and 4:1 or less, 3:1 or less, 2.5:1 or less, 2:1 or less, 1.8:1 or less, or 1.5:1 or less.

When it is desired to evaluate the biodegradability of biodegradable resin in soil and compost environments, the enzyme unit ratio of lipase:cutinase may be 0.5:1 or more, or 0.8:1 or more, and 2:1 or less, 1.8:1 or less, or 1.5:1 or less.

Specifically, when it is desired to evaluate the biodegradability of biodegradable resin in soil and compost environments, the amount of lipase per 1 unit of cutinase may be 0.5 unit or more, or 0.8 unit or more, and 2 unit or less, or 1.8 unit or less, or 1.5 unit or less.

Meanwhile, when additional enzymes are included during soil and compost environment simulation, $\alpha$-amylase is 100 unit or more, or 200 unit or more, and 3,000 unit or less, or 1,500 unit or less; cellulase is 700 unit or more, or 1,400 unit or more, and 3,400 unit or less, or 1,700 unit or less; proteinase K, protease, and alcalase may each be 0.75 unit or more, or 1.5 unit or more, and 5 unit or less, or 2.5 unit or less.

When it is desired to evaluate the biodegradability of biodegradable resin in a marine environment, the cutinase may be 10 unit or more, or 20 unit or more, or 100 unit or more, 200 unit or more, or 500 unit or more, or 1,000 unit or more, or 2,000 unit or more, and 40,000 unit or less, or 20,000 unit or less, or 16,000 unit or less, or 10,000 unit or less, or 8,000 unit or less, or 6,000 unit or less, or 3,000 unit or less, and the lipase may be 10 unit or more, or 20 unit or more, or 800 unit or more, or 1,500 unit or more, or 1,600 unit or more, or 3,000 unit or more, and 40,000 unit or less, or 30,000 unit or less, or 20,000 unit or less, or 15,000 unit or less, or 10,000 unit or less, or 8,000 unit or less, or 4,000 unit or less.

When it is desired to evaluate the biodegradability of biodegradable resin in a marine environment, the enzyme unit ratio of lipase:cutinase may be 0.5:1 or more, 0.8:1 or more, or 1:1 or more, and 4:1 or less, 3:1 or less, 2.5:1 or less.

Specifically, when it is desired to evaluate the biodegradability of biodegradable resin in a marine environment, the amount of lipase per 1 unit of cutinase may be 0.5 unit or more, 0.8 unit or more, or 1 unit or more, or 1.5 unit or more, and 4 unit or less, or 3 unit or less, or 2.5 unit or less.

When additional enzymes are included during marine environment simulation, $\alpha$-amylase is 100 unit or more, or 200 unit or more, and 3,000 unit or less, or 1,500 unit or less; cellulase is 700 unit or more, or 1,400 unit or more, and 3,400 unit or less, or 1,700 unit or less; proteinase K, protease, and alcalase may each be 0.75 unit or more, or 1.5 unit or more, and 5 unit or less, or 2.5 unit or less.

The unit "unit" of the hydrolase is an enzyme unit or an international unit of enzyme activity, also denoted as U or I.U. One unit of enzyme means the amount of enzyme that converts 1 $\mu$mol of substrate into a product in 1 minute under the optimum reaction conditions (temperature, pH, substrate concentration) of the corresponding enzyme.

The optimal reaction conditions differ depending on the enzyme. For example, the optimal reaction temperature of the aforementioned lipase, cutinase, and additional enzymes is in the range of 25 °C to 58 °C. The pH is in the range of 4 to 9, and may vary depending on conditions (for example, soil conditions 4 to 9, marine conditions 7.9 to 8.2, compost conditions 6 to 9).

**Biodegradability evaluation method for biodegradable resin**

According to an embodiment of the present disclosure, there is provided a biodegradability evaluation method for biodegradable resin comprising the steps of: i) contacting an enzyme solution comprising a hydrolase comprising lipase and cutinase, and phosphate-buffered saline with a biodegradable resin; and ii) observing a degradation state of the biodegradable resin over time.

Biodegradation of biodegradable resin is a process in which a polymer is hydrolyzed into oligomers or monomers, and then the oligomers or monomers are metabolized by microorganisms. Therefore, the faster the rate of hydrolysis, which is the first step, the faster biodegradation occurs.

The evaluation method of the present disclosure is focused on this point, and according to the evaluation method, relative biodegradability of biodegradable resin can be evaluated easily and quickly by measuring the hydrolysis rate of biodegradable resin using an enzyme solution comprising a hydrolase comprising lipase and cutinase.

Also, various environments such as soil, compost, and marine can be simulated by changing the composition of the enzyme included in the enzyme solution. Accordingly, according to the biodegradability evaluation method, relative biodegradability when biodegradable resin is exposed to natural environments of various conditions can be predicted quickly and easily.

The biodegradability evaluation method for biodegradable resin according to an embodiment of the present disclosure can be applied to various resins requiring biodegradability evaluation. Examples of biodegradable resin that can be evaluation targets include polylactide (PLA), poly(butylene adipate-co-terephthalate) (PBAT), polybutylene succinate (PBS), polycaprolactone (PCL), polyhydroxybutyric acid (PHB), thermoplastic starch, and the like, but are not limited thereto, and relative biodegradation rates in natural environments can be predicted easily and quickly by applying the biodegradability evaluation method of the present disclosure to any resin expected to have biodegradability.

According to the biodegradability evaluation method, it is possible to evaluate the biodegradability of a resin within several hours to several days. Accordingly, the biodegradability evaluation method can be usefully used when a simple biodegradability test is required in a short period of time, for example, in the development stage of biodegradable resin.

The temperature and pressure conditions of the biodegradability evaluation method for biodegradable resin can be appropriately selected according to the natural environment conditions to be simulated. As an example, the biodegradability evaluation method for biodegradable resin of the present disclosure may be performed under normal pressure (760±20 torr) in a temperature range of 20 °C to 30 °C, or 30 °C to 40 °C, or 40 °C to 60 °C.

The composition of the enzyme solution used in the biodegradability evaluation method for biodegradable resin is as described above.

Specifically, the biodegradability evaluation method for biodegradable resin may be performed as in Method 1 to Method 3 below.

### a. Method 1

In Method 1, step i) may be performed by dropping the enzyme solution onto a resin specimen comprising biodegradable resin.

Also, step ii) may be performed by measuring a change in decomposition area of the resin specimen over time.

Specifically, Method 1 may include: preparing an enzyme solution comprising lipase and cutinase; dropping the enzyme solution onto a resin specimen comprising biodegradable resin; and measuring a change in decomposition area of the resin specimen over time.

The resin specimen includes biodegradable resin requiring biodegradability measurement, and its shape is not limited, but a two-dimensional plate-like shape such as a film or sheet is preferable because the degradation state after dropping the enzyme solution can be easily identified.

The resin specimen may consist only of biodegradable resin or a biodegradable resin compound requiring biodegradability measurement.

The size of the film or sheet-type resin specimen is not limited, but for example, it may be preferable that the width and length are 5 cm or more, or 10 cm or more, and in a range of 20 cm or less, respectively.

The film or sheet-type resin specimen may preferably have a thickness of 250 $\mu$m or less, or 100 $\mu$m or less, and 20 $\mu$m or more, or 50 $\mu$m or more.

After preparing the enzyme solution and the resin specimen as described above, the enzyme solution is dropped on the resin specimen, and the biodegradability is evaluated by observing the degradation state of the resin specimen over time.

The method of dropping the enzyme solution onto the resin specimen is not particularly limited, but it is preferable to drop it on at least one or more points on the surface of the specimen using a pipet or the like, and the amount of enzyme solution dropped on one point is set to a level of 3 to 5 $\mu$l. At this time, in order to reduce the error of the biodegradability evaluation, two or more points for dropping the enzyme solution may be used.

The points for dropping the enzyme solution are separated from the edge of the specimen by at least 20 mm, and when dropping on two or more points, a distance of at least 10 mm is secured between each point so that the collapse points generated by the decomposition of the resin during the evaluation process do not overlap each other.

The biodegradability evaluation of biodegradable resin is performed by measuring the area of collapse generated on the specimen over time from immediately after dropping the enzyme solution on the specimen as described above.

In general, a resin with a faster hydrolysis rate has higher biodegradability, and the generation and expansion of the collapse area occur rapidly during the biodegradability evaluation. Therefore, relative biodegradability of each resin in a natural environment can be predicted by testing two or more biodegradable resins simultaneously using the method.

Temperature and pressure conditions during dropping of the enzyme solution and measurement of the decomposition area can be appropriately selected according to natural environmental conditions to be simulated. As an example, the biodegradability evaluation method for biodegradable resin of the present disclosure may be performed under normal pressure (760±20 torr) in a range of 20 °C to 60 °C, for example, in a temperature range of 20 °C to 30 °C, or 30 °C to 40 °C, or 40 °C to 60 °C.

According to the biodegradability evaluation method for biodegradable resin, the biodegradability of a resin can be evaluated within several hours to several days. The evaluation time may vary depending on the size and weight of the biodegradable resin specimen, the composition of the enzyme solution, etc., but does not exceed a maximum of 14 days.

### b. Method 2

In Method 2, step i) may be performed by immersing a resin specimen comprising biodegradable resin in the enzyme solution.

Specifically, Method 2 may include: preparing an enzyme solution comprising lipase and cutinase; immersing a resin specimen comprising biodegradable resin in the enzyme solution; and observing a degradation state of the immersed resin specimen over time.

The resin specimen includes biodegradable resin requiring biodegradability measurement, and its shape is not limited, and various shapes such as a film type and a sheet type can be used. Among these, the film type is more preferable because the degradation state can be quickly identified when immersed in a hydrolysis solution.

In this aspect, the film-type specimen may preferably have a thickness of 250 $\mu$m or less, or 100 $\mu$m or less, and 20 $\mu$m or more, or 50 $\mu$m or more.

After preparing the enzyme solution and the resin specimen as described above, the resin specimen is immersed in the enzyme solution, and the biodegradability is evaluated by observing the degradation state of the immersed resin specimen over time.

The amount of the enzyme solution used is not limited and is used in an amount such that the resin specimen can be completely immersed.

That is, the amount of the enzyme solution used can be appropriately adjusted according to the concentration of the enzyme solution and the size and/or weight of the resin specimen. For example, when using an enzyme solution containing 4,000 to 40,000 unit of hydrolase per 1 ml, the enzyme solution can be used in an amount of 100 $\mu$l or more, or 250 $\mu$l or more, and 1000 $\mu$l or less, or 750 $\mu$l or less, or 500 $\mu$l or less per 10 x 10 mm2 of a resin specimen having a thickness of 20 to 250 $\mu$m. However, the amount of enzyme solution used is not limited thereto, and an appropriate amount is selected and used according to the concentration of hydrolase in the hydrolase, or the shape, size, weight, etc., of the resin specimen.

Since the biodegradable resin specimen is hydrolyzed in the enzyme solution over time, the weight of the specimen gradually decreases, and it can be visually confirmed that the specimen collapses and is divided into several pieces. Accordingly, the 'observation of degradation state' can be performed by various methods of observing changes over time, such as measuring the weight change of the resin specimen at regular intervals or visually observing the collapse of the resin specimen.

Relative biodegradability of each resin in a natural environment can be predicted by testing two or more biodegradable resins simultaneously by the above method. In general, a resin with a faster hydrolysis rate has higher biodegradability, and thus biodegradability can be relatively evaluated by comparing the hydrolysis rates of two or more biodegradable resins.

Temperature and pressure conditions of the step of immersing the resin specimen in the enzyme solution and the step of observing the degradation state of the resin specimen can be appropriately selected according to natural environmental conditions to be simulated. As an example, the biodegradability evaluation method for biodegradable resin of the present disclosure may be performed under normal pressure (760$\pm$20 torr) in a range of 20 °C to 60 °C, for example, in a temperature range of 20 °C to 30 °C, or 30 °C to 40 °C, or 40 °C to 60 °C.

According to the biodegradability evaluation method for biodegradable resin, the biodegradability of a resin can be evaluated within several hours to several days. The evaluation time may vary depending on the size and weight of the biodegradable resin specimen, the composition of the enzyme solution, etc., but does not exceed a maximum of 14 days.

### c. Method 3

In Method 3, step i) is performed by spraying a biodegradable resin solution onto a medium to prepare a specimen, and then dropping the enzyme solution onto the specimen, and

step ii) may be performed by observing a change in a clear zone formed on the specimen over time.

Specifically, Method 3 may include: preparing a specimen by spraying a biodegradable resin solution onto a medium; dropping an enzyme solution comprising lipase and cutinase onto the specimen; and measuring a change in a clear zone formed on the specimen over time.

The medium is a means for supporting a resin solution comprising biodegradable resin and providing a moist environment in which enzymes can function, and is preferably a material non-reactive to the biodegradable resin and the enzyme solution.

The material of the medium can be selected depending on the type of biodegradable resin and the composition of the enzyme included in the enzyme solution. For example, the medium may be a solid medium including agar and/or gelatin, but is not limited thereto.

Unlike a medium for culturing microorganisms, the medium may consist only of agar and/or gelatin and distilled water, without additives such as sugars and peptides.

Such a medium is preferably sterilized before use. As an example, the medium can be sterilized by treatment for 10 minutes to 60 minutes under conditions of a temperature of 100 °C to 150 °C and a pressure of 0.05 MPa to 0.3 MPa. However, the sterilization method of the medium is not limited thereto, and a sterilization method known for each material can be appropriately selected.

The biodegradable resin solution is composed of biodegradable resin, which is a target for biodegradability evaluation, and a solvent.

The solvent is not particularly limited as long as it can uniformly disperse the biodegradable resin, and as an example, at least one selected from the group consisting of chloroform, methyl chloride, carbon tetrachloride, benzene, dimethyl sulfoxide, dimethylformamide, toluene, and xylene can be used.

The content (solid content) of the biodegradable resin in the biodegradable resin solution may be 0.5% by weight or more, or 1% by weight or more, and in a range of 3% by weight or less, or 2% by weight or less. When the range is satisfied, droplets of the biodegradable resin solution can be uniformly applied at an appropriate concentration onto the medium, and accordingly, an environment favorable for the hydrolase to act can be created, which is preferable.

A method of spraying the biodegradable resin solution is not particularly limited, and a conventionally known spraying method of a polymer solution can be appropriately selected. For example, a spraying device such as a nebulizer, a sprayer, an airbrush, or an electric spraying device can be used.

After spraying the biodegradable resin solution onto the medium, it is dried so that all the solvents in the biodegradable resin solution can be volatilized. If the biodegradability evaluation is performed without the solvent being volatilized, the activity of the enzyme may be very low, making smooth evaluation difficult. Therefore, drying is performed by adjusting temperature, pressure, and time in consideration of the solvent used, the type of biodegradable resin, and characteristics of the medium. Taking the case of using chloroform as a solvent as an example, the medium on which the biodegradable resin solution is sprayed can be dried by leaving it for 5 minutes to 20 minutes, preferably 10 minutes to 20 minutes, under room temperature (25°C) and normal pressure (0.1MPa).

The spraying of the biodegradable resin solution can be performed so that the average thickness of the biodegradable resin applied on the medium is 0.6 $\mu$m or more, or 0.8 $\mu$m or more, and in a range of 1.5 $\mu$m or less. When the biodegradable resin is applied at an appropriate thickness as described above, it is suitable for observing changes over time after dropping the enzyme solution.

Next, the biodegradability evaluation is started by dropping an enzyme solution comprising lipase and cutinase as hydrolases onto the medium (specimen) on which the biodegradable resin solution has been sprayed.

At this time, various environments such as soil environment, compost environment, and marine environment can be implemented by adjusting the type and/or content of the hydrolase included in the enzyme solution. The enzyme composition of the enzyme solution for simulating each condition is as described above.

The method of dropping the enzyme solution onto the medium (specimen) on which the biodegradable resin solution is sprayed is not particularly limited, but it is preferable to drop it on at least one or more points on the surface of the specimen (the surface on which the biodegradable resin solution is applied) using a pipet or the like, and the amount of enzyme solution dropped on one point is set to a level of 3 $\mu$l to 5 $\mu$l. At this time, in order to reduce the error of the biodegradability evaluation, two or more points for dropping the enzyme solution may be used.

The position for dropping the enzyme solution on the specimen is not particularly limited, but preferably, it is separated from the edge of the specimen by at least 20 mm, and when dropping on two or more points, a distance of at least 20 mm is secured between each point so that clear zones generated by the decomposition of the resin during the evaluation process do not overlap each other. Depending on the sample, a faint clear zone may be formed widely. When using such a sample, the enzyme solution is dropped so as to be separated from the edge of the specimen by at least 40 mm, and when dropping on two or more points, a distance of at least 40 mm is secured between each point so that the clear zones do not overlap each other. In this way, the dropping point of the enzyme solution on the specimen can be determined in consideration of the characteristics of the sample.

The clear zone is a transparent part produced by the decomposition of water-insoluble biodegradable resin into water-soluble molecules by hydrolase. The biodegradability evaluation of biodegradable resin is performed by measuring the change in the area of the clear zone or the difference in the degree to which the clear zone becomes transparent over time from immediately after dropping the enzyme solution on the specimen as described above.

In general, a resin with a faster hydrolysis rate has higher biodegradability, and the generation and expansion of the clear zone occur rapidly during the biodegradability evaluation. Therefore, relative biodegradability of each resin in a natural environment can be predicted by testing two or more biodegradable resins simultaneously using the method.

Temperature and pressure conditions during dropping of the enzyme solution and measurement of the decomposition area can be appropriately selected according to natural environmental conditions to be simulated. As an example, the biodegradability evaluation method for biodegradable resin of the present disclosure may be performed under normal pressure (760±20 torr) in a temperature range of 20 °C to 30 °C, or 30 °C to 40 °C, or 40 °C to 60 °C.

According to the biodegradability evaluation method for biodegradable resin, the biodegradability of a resin can be evaluated within several hours to several days. The evaluation time may vary depending on the solid content of the biodegradable resin solution, the composition of the enzyme solution, etc., but does not exceed a maximum of 7 days.

### d. Image analysis of biodegradable resin specimen

Meanwhile, in the biodegradability evaluation method for biodegradable resin, the step of ii) observing a degradation state of the biodegradable resin over time may include: a) obtaining an image of the resin specimen according to elapsed unit time; and b) evaluating biodegradability through a change in the image of the resin specimen over time.

First, images of the biodegradable resin specimen in contact with the enzyme solution are acquired over a certain time (unit time). The unit time may be, for example, about several seconds, several minutes, or several hours, and this can be set differently depending on the composition of the specimen or hydrolase.

If method 1 or 3 is followed, it may be preferable that step a) is performed under conditions to prevent evaporation of the dropped enzyme solution. When taking images, it is preferable to maintain the same color balance, illuminance, shutter time, lens brightness, and other shooting conditions to increase the accuracy of the analysis.

In this case, a real-time image measurement device or the like can be used for the image, and for example, a device such as JuLI™ Stage of NanoEntek can be used, but the present disclosure is not necessarily limited thereto.

Next, biodegradability is evaluated through the image change of the resin specimen over time. As time passes, in the resin specimen in contact with the enzyme solution, hydrolysis of the resin by hydrolase occurs, and accordingly, an appearance change occurs.

For example, in a region where hydrolysis occurs, the resin thickness of the specimen becomes thin, and a brighter region (Clear Zone) may be formed in appearance compared to a region where hydrolysis does not occur.

Therefore, by measuring and analyzing the change in this bright region, the degree of biodegradation of the resin in the corresponding specimen can be confirmed.

However, in order to accurately analyze this, it is necessary to quantify and standardize the shape of the bright region.

According to an example, step b) may include: b-1) for each image of the resin specimen obtained according to elapsed unit time, obtaining a brightness difference with the image immediately after dropping the hydrolase; b-2) representing image brightness difference values as a function of elapsed unit time; and b-3) evaluating biodegradability through analysis of the function.

That is, in the obtained image, the brightness of the area where the hydrolase is dropped is measured, and the change in brightness is confirmed over time. Then, the difference between the brightness measured at a specific elapsed time and the brightness immediately after dropping the hydrolase is obtained.

If this brightness difference is compared in the form of a function over time, the degree of brightness change over time can be confirmed. And, depending on the pattern of the brightness change, it can be assumed that a specific point in time, for example, the time when the second derivative of the function is at its maximum, is the time when the acceleration of biodegradation of the biodegradable resin reached its maximum value and at the same time is the time when the clear zone began to form.

The time when the second derivative of the function is maximum is a kind of inflection point in the brightness function according to the elapsed time, and is a point where the speed of brightness change changes, which can be seen as a point where the biodegradation speed changes. Therefore, if the 'elapsed time' at the point where the 'second derivative is maximum' of the function is relatively early, the biodegradation rate is relatively fast, and if the 'elapsed time' at the point where the 'second derivative is maximum' of the function is relatively slow, the biodegradation rate is relatively slow, and thus the biodegradability of the resin can be evaluated.

Hereinafter, embodiments of the present disclosure will be described in more detail in the following examples. However, the following examples only illustrate embodiments of the present disclosure, and the contents of the present disclosure are not limited by the following examples.

[Examples]

## Example A-1: Biodegradability evaluation in a soil/compost environment

(1) Preparation of specimen

Biodegradable resin was put into a twin-screw extruder (die diameter = 32 mm) and extruded under conditions of Barrel Temperature of 180°C to 200°C, Feed Rate of 30 to 50 kg/hr, and 300 rpm, to prepare a resin composition in a pellet form.

Thereafter, the pellet-shaped resin composition was molded to a thickness of 0.05 mm at an extrusion temperature of 170°C using a single-screw extruder (Blown Film M/C, 19 pi, L/D=25) to prepare a blown film. At this time, the Blown-Up Ratio was 1.8 and the line speed was 5 m/min.

Thereafter, it was cut into a film type of width * length = 5 cm * 5 cm and a thickness of 0.05 mm, and then inserted into a fixing frame and used as a specimen.

Specimens of each of the following polymer compounds were prepared by the above method.

a. Poly(butylene adipate-co-terephthalate) compound 1 (Manufacturer: LG Chem, raw materials: PBAT and PLA)
b. Poly(butylene adipate-co-terephthalate) compound 2 (Manufacturer: LG Chem, raw materials: PBAT, PLA, and additives)

(2) Preparation of enzyme solution

An enzyme solution was prepared by mixing 2 types of lipase and 2 types of cutinase below with 1 ml of 1X Phosphate-Buffered Saline (PBS).

Lipase *Rhizopus Oryzae* 10,000 unit
Lipase *Pseudomonas Cepacia* 10,000 unit
Cutinase *Aspergillus Oryzae* 10,000 unit
Cutinase *Humicola Insolens* 10,000 unit

The PBS is a mixture of ultrapure grade phosphate buffer adjusted to pH 7.4 and saline containing 137 mM NaCl, 2.7 mM KCl, 8 mM $Na_2HPO_4$, and 2 mM $KH_2PO_4$, and PBS of the same composition was used in all the following examples.

(3) Biodegradability evaluation

Biodegradability evaluation was performed by the following method under conditions of 58 °C and 760 mmHg.

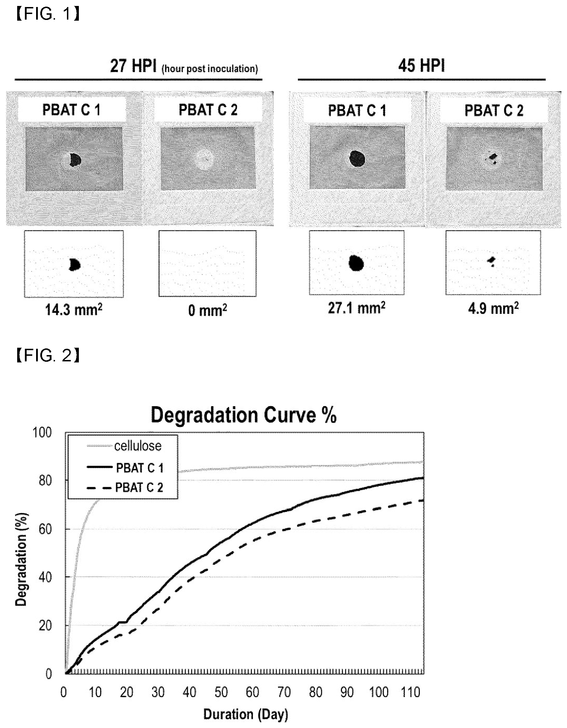

5 μl of the enzyme solution of (2) was dropped onto the specimen prepared in (1). After 27 hours and 45 hours, the size of the lost area generated by the decomposition of the specimen by the enzyme solution was measured (FIG. 1).

(4) Result analysis

FIG. 2 shows the biodegradability test results under industrial composting conditions of poly(butylene adipate-co-terephthalate) compound 1 (PBAT C1), poly(butylene adipate-co-terephthalate) compound 2 (PBAT C2), and cellulose (manufacturer: Sigma-Aldrich, product number: 310697) measured according to the ISO 14855-1 standard. Referring to FIG. 2, it can be confirmed that the biodegradability of PBAT C1 not containing additives is higher than that of PBAT C2.

FIG. 1 is the biodegradability evaluation result of (3), and the first row is photos taken when 27 hours elapsed (27HPI) and 45 hours elapsed (47HPI) after dropping the enzyme solution on the specimen. The second row of FIG. 1 is photos showing only the lost part in the photos of the first row in order to confirm the area of the specimen lost by the hydrolase, and the lost area is described below.

Referring to FIG. 1, it can be confirmed that the biodegradability of PBAT C1 not containing additives is higher than that of PBAT C2, which is consistent with the results in FIG. 2 above.

**Example A-2: Biodegradability evaluation in a marine environment**

(1) Preparation of specimen

Biodegradable resin was put into a twin-screw extruder (die diameter = 32 mm) and extruded under conditions of Barrel Temperature of 180°C to 200°C, Feed Rate of 30 to 50 kg/hr, and 300 rpm, to prepare a resin composition in a pellet form.

Thereafter, the pellet-shaped resin composition was molded to a thickness of 0.05 mm at an extrusion temperature of 170 °C using a single-screw extruder (Single Screw Extruder, Blown Film M/C, 19 pi, L/D=25) to prepare a blown film. At this time, the Blown-Up Ratio was 1.8 and the line speed was 5 m/min.

Thereafter, it was cut into a width * length = 1 cm * 1 cm and a thickness of 0.05 mm to prepare a film-type specimen.

Specimens of each of the following polymers were prepared by the above method.

a. Poly(butylene succinate) (Manufacturer: PTT MCC, raw material: PBS)
b. Poly(butylene succinate adipate) (Manufacturer: LG Chem, raw material: PBSA)

(2) Preparation of enzyme solution

An enzyme solution was prepared by mixing 1 type of lipase and 2 types of cutinase below with 1 ml of 1X Phosphate-Buffered Saline (PBS) and 36 mg of sea salt.

Lipase *Pseudomonas Cepacia* 10,000 unit
Cutinase *Aspergillus Oryzae* 10,000 unit
Cutinase *Humicola Insolens* 10,000 unit

(3) Biodegradability evaluation

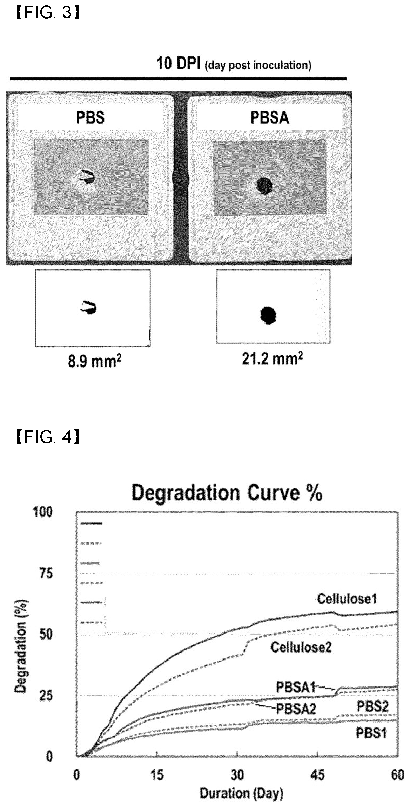

Biodegradability evaluation was performed by the following method under conditions of 30 °C and 760 mmHg.
5 $\mu$l of the enzyme solution of (2) was dropped onto the specimen prepared in (1). After 7 days, the size of the lost area generated by the decomposition of the specimen by the enzyme solution was measured (FIG. 3).

(4) Result analysis

FIG. 3 shows the biodegradability evaluation results of (3). In FIG. 3, DPI (day post-inoculation) means the number of days elapsed, and 10 DPI means that it is a photo 10 days after dropping the enzyme solution on the specimen.
Table 1 and FIG. 4 below show the results of the biodegradability test under marine conditions measured according to the ASTM D6691 standard for the polymers of a. and b. above. The test was repeated twice for each specimen, and the test count was described as "n" after each legend. That is, "Cellulose1" and "Cellulose2" are experimental results performed on the same cellulose, respectively, where "Cellulose1" means the first experimental result and "Cellulose2" means the second experimental result. The values in Table 1 mean the decomposition rate (%) of the corresponding resin after each day elapsed.

[Table 1]

| Elapsed Days | Cellulose1 | Cellulose2 | PBS1 | PBS2 | PBSA1 | PBSA2 |
|---|---|---|---|---|---|---|
| 15 | 36.7 | 28.7 | 9.2 | 10.6 | 17.6 | 15.7 |
| 30 | 52.1 | 41.3 | 11.5 | 13.2 | 22.9 | 21.4 |
| 45 | 58.4 | 52.9 | 13.8 | 15.1 | 24.5 | 24.4 |
| Final(60) | 59.2 | 54.0 | 14.7 | 17.3 | 28.7 | 27.7 |

The first row of FIG. 3 is a photo taken 10 days after dropping the enzyme solution on the specimen (10 DPI). The second row of FIG. 3 is a photo showing only the lost part in the photo of the first row to confirm the area of the specimen lost by the hydrolase, and the lost area is described below.
Referring to FIG. 3, it can be confirmed that the biodegradability of PBSA is higher than that of PBS, which is consistent with the results in FIG. 4 above.

**Example B-1: Biodegradability evaluation in a soil/compost environment**

(1) Preparation of specimen

Biodegradable resin was put into a twin-screw extruder (die diameter = 32 mm) and extruded under conditions of Barrel Temperature of 180°C to 200°C, Feed Rate of 30 to 50 kg/hr, and 300 rpm, to prepare a resin composition in a pellet form.
Thereafter, the pellet-shaped resin composition was molded to a thickness of 0.05 mm at an extrusion temperature of 170 °C using a single-screw extruder (Single Screw Extruder, Blown Film M/C, 19 pi, L/D=25) to prepare a blown film. At this time, the Blown-Up Ratio was 1.8 and the line speed was 5 m/min.
Thereafter, it was cut into a width * length = 1 cm * 1 cm and a thickness of 0.05 mm to prepare a film-type specimen.
Specimens of each of the following polymer compounds were prepared by the above method.

a. Poly(butylene adipate-co-terephthalate) / thermoplastic starch compound (LG Chem, PBAT/TPS Comp'd)
b. Poly(butylene adipate-co-terephthalate) / polylactic acid compound (LG Chem, PBAT/PLA Comp'd)

(2) Preparation of enzyme solution

An enzyme solution was prepared by mixing 2 types of lipase, 2 types of cutinase, and 1 type of alpha-amylase below with 1 ml of 1X Phosphate-Buffered Saline (PBS).

Lipase *Rhizopus Oryzae* 1000 unit
Lipase *Pseudomonas Cepacia* 3000 unit
Cutinase *Aspergillus Oryzae* 2000 unit
Cutinase *Humicola Insolens* 1500 unit
$\alpha$-amylase *Bacillus Licheniformis* 1500 unit

(3) Biodegradability evaluation

Biodegradability evaluation was performed by the following method under conditions of 37 °C and 760 mmHg.
0.5 ml of the enzyme solution of (2) was put into one well of 24-well plates, and two sheets of the specimen of (1) were immersed. Thereafter, the pattern in which the specimen collapsed and pieces fell off was visually observed every 1 to 3 days.

(4) Result analysis

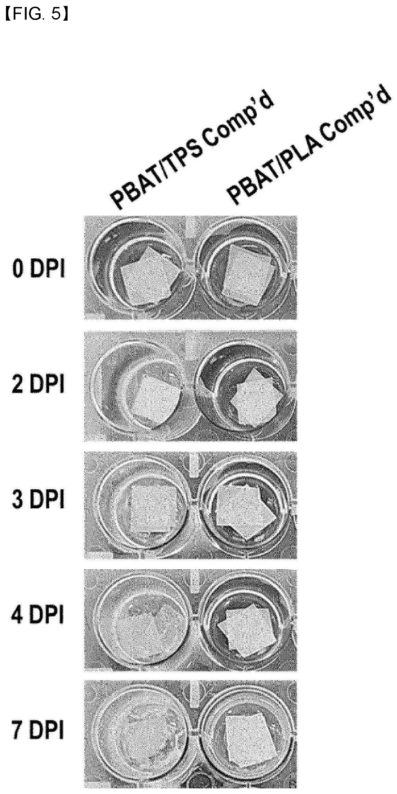

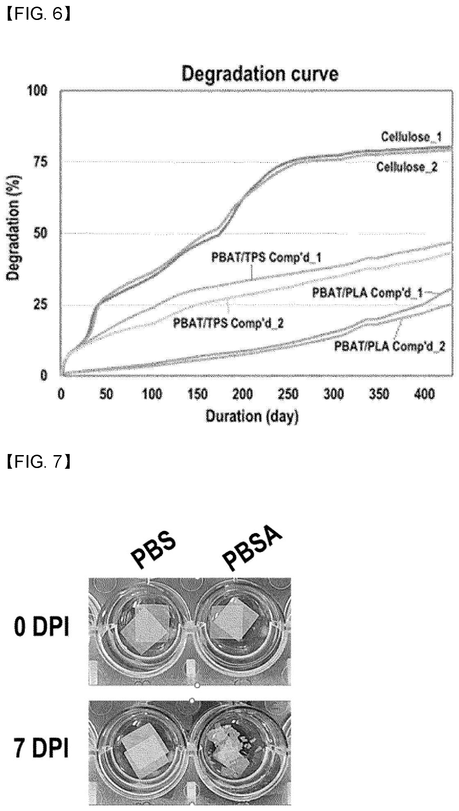

FIG. 5 shows the biodegradability evaluation results of (3). In FIG. 5, DPI (day post-inoculation) means the number of days elapsed, 0 DPI is a photo immediately after immersing the specimen in the enzyme solution, and 2 DPI is a photo 2 days after immersion.
Table 2 and FIG. 6 below show the results of the biodegradability test under soil conditions measured according to the ISO 17556 standard for the polymer compounds of a. and b. above. The test was repeated twice for each specimen, and the test count was described as "_n" after each legend. That is, "Cellulose_1" and "Cellulose_2" are experimental results performed on the same cellulose, respectively, where "Cellulose_1" means the first experimental result and "Cellulose_2" means the second experimental result. The values in Table 2 mean the decomposition rate (%) of the corresponding resin after each day elapsed.

[Table 2]

| Elapsed Days | Cellulose_1 | Cellulose_2 | PBAT/TPS comp'd_1 | PBAT/TPS comp'd_2 | PBAT/PLA comp'd_1 | PBAT/PLA comp'd_2 |
|---|---|---|---|---|---|---|
| 45 | 25.7 | 26.4 | 14.8 | 13.2 | 2.2 | 1.8 |
| 90 | 32.9 | 34.7 | 22.4 | 17.5 | 3.7 | 3.0 |
| 180 | 52.0 | 54.5 | 31.9 | 26.9 | 7.8 | 6.6 |
| 360 | 79.1 | 77.8 | 42.2 | 38.3 | 21.0 | 19.0 |
| Final(430) | 80.2 | 79.1 | 46.7 | 43.1 | 30.5 | 25.0 |

Referring to FIG. 5, in a. PBAT/TPS compound, it can be confirmed that small holes were made and it started to collapse after 4 days of immersion, and a considerable part of the edge of the specimen collapsed on the 7th day. On the other hand, in b. PBAT/PLA compound, since the specimen did not collapse clearly even on the 7th day of immersion, it can be confirmed that a. PBAT/TPS compound has relatively high biodegradability compared with b. PBAT/PLA compound, which is consistent with the results in Table 2 and FIG. 6 measured according to the standard test method.

**Example B-2: Biodegradability evaluation in a marine environment**

(1) Preparation of specimen

Biodegradable resin was put into a twin-screw extruder (die diameter = 32 mm) and extruded under conditions of Barrel Temperature of 180°C to 200°C, Feed Rate of 30 to 50 kg/hr, and 300 rpm, to prepare a resin composition in a pellet form.
Thereafter, the pellet-shaped resin composition was molded to a thickness of 0.05 mm at an extrusion temperature of 170 °C using a single-screw extruder (Single Screw Extruder, Blown Film M/C, 19 pi, L/D=25) to prepare a blown film. At this time, the Blown-Up Ratio was 1.8 and the line speed was 5 m/min.

Thereafter, it was cut into a width * length = 1 cm * 1 cm and a thickness of 0.05 mm to prepare a film-type specimen.

Specimens of each of the following polymers were prepared by the above method.

    a. Poly(butylene succinate) (Manufacturer: PTT MCC, raw material: PBS)
    b. Poly(butylene succinate adipate) (Manufacturer: LG Chem, raw material: PBSA)

(2) Preparation of enzyme solution

An enzyme solution was prepared by mixing 1 type of lipase and 2 types of cutinase below with 1 ml of 1X Phosphate-Buffered Saline (PBS) and 36 mg of sea salt.

    Lipase *Pseudomonas Cepacia* 10,000 unit
    Cutinase *Aspergillus Oryzae* 10,000 unit
    Cutinase *Humicola Insolens* 10,000 unit

(3) Biodegradability evaluation

Biodegradability evaluation was performed by the following method under conditions of 30 °C and 760 mmHg.
0.5 ml of the enzyme solution of (2) was put into one well of 24-well plates, and two sheets of the specimen of (1) were immersed. Thereafter, after 7 days, the pattern in which the specimen collapsed and pieces fell off was visually observed.

(4) Result analysis

FIG. 7 shows the biodegradability evaluation results of (3). In FIG. 7, DPI (day post-inoculation) means the number of days elapsed, 0 DPI is a photo immediately after immersing the specimen in the enzyme solution, and 7 DPI is a photo 7 days after immersion.

Referring to FIG. 7, it can be confirmed that a considerable part of the specimen of b. poly butylene succinate adipate (PBSA) collapsed after 7 days of immersion. On the other hand, in a. poly butylene succinate (PBA), since the specimen did not collapse clearly, it can be confirmed that b. poly butylene succinate adipate (PBSA) has relatively high biodegradability compared with a. poly butylene succinate (PBA). This is consistent with the results in Table 1 and FIG. 4 measured according to the standard test method.

**Example C-1, Comparative Example C-1, and Comparative Example C-2: Biodegradability evaluation in a soil/ compost environment**

(1) Preparation of medium

A circular 1% agar plate with a diameter of 90 mm was prepared by a method of preparing a Petri dish medium.
A solution was prepared by adding 10 g of agar (Manufacturer: BD Difco, product name: Bacto Agar, product number: 214010) and 1000 ml of distilled water to a 2000 ml Erlenmeyer flask. The solution was put into an autoclave and sterilized at a temperature of 121°C and a pressure of 0.1 MPa for 15 minutes. The sterilized solution was dispensed into sterile circular Petri dishes with a diameter of 90 mm in 10 ml portions to prepare a medium.

(2) Preparation of resin solution

1) Preparation of PBAT compound 1 solution

PBAT compound 1 was prepared by mixing 79.2% by weight of poly-butylene adipate-co-terephthalate (PBAT, LG Chem); 8.8% by weight of polylactic acid (PLA, TotalEnergies Corbion); and 12% by weight of calcium carbonate ($CaCO_3$). Each of the resins used was disinfected with 70% ethanol.
The PBAT compound 1 was dissolved in 20 ml of chloroform at 1% by weight to prepare a PBAT compound 1 solution.

2) Preparation of PBAT compound 2 solution

PBAT compound 2 was prepared by mixing 79.2% by weight of poly-butylene adipate-co-terephthalate (PBAT, LG Chem); 8.8% by weight of meso-polylactic acid (meso-PLA, Bio Valore); and 12% by weight of calcium carbonate ($CaCO_3$). Each of the resins used was disinfected with 70% ethanol.

The PBAT compound 2 was dissolved in 20 ml of chloroform at 1% by weight to prepare a PBAT compound 2 solution.

(3) Preparation of enzyme solution

Enzyme solutions (stock solutions) of the following Example C-1, Comparative Example C-1, and Comparative Example C-2 were prepared. In addition, 1/10 dilutions of the stock solutions were prepared.

<Example C-1>

1X Phosphate-Buffered Saline (PBS) 1 ml
Lipase *Rhizopus Oryzae* 1000 unit
Lipase *Pseudomonas Cepacia* 3000 unit
Cutinase *Aspergillus Oryzae* 2000 unit
Cutinase *Humicola Insolens* 1500 unit

<Comparative Example C-1>

1X Phosphate-Buffered Saline (PBS) 1 ml
Cutinase *Humicola Insolens* 1500 unit

<Comparative Example C-2>

1X Phosphate-Buffered Saline (PBS) 1 ml
Lipase *Pseudomonas Cepacia* 3000 unit

(4) Biodegradability evaluation

On the agar plate of (1), 6 ml of the PBAT compound 1 solution prepared in 1) of (2) was evenly sprayed and dried at room temperature (25°C) for about 10 minutes so that the solvent was volatilized, thereby preparing a "PBAT compound 1" specimen having an average thickness of PBAT compound 1 of 0.7 $\mu$m. The average thickness of the PBAT compound 1 was obtained by measuring the roughness of the specimen before and after spraying the PBAT compound 1 solution with an optical profiler (model name: NewView™ 8300, Zygo corp.).

In the same manner, on a separate agar plate, the PBAT compound 2 solution prepared in 2) of (2) was sprayed and dried to prepare a "PBAT compound 2" specimen having an average thickness of PBAT compound 2 of 0.7 $\mu$m.

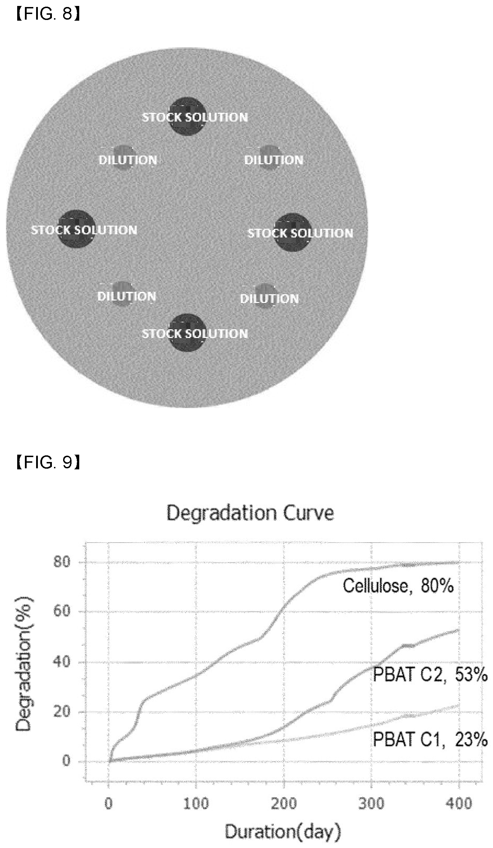

On each of the specimens, as shown in FIG. 8, the enzyme solution stock solution and 1/10 dilution of Example C-1 of (3) were dropped at four points each in 5 $\mu$l portions. Thereafter, the area and clarity of the clear zone generated by decomposition of the resin sprayed on the medium by the enzyme solution were visually measured over time.

For the enzyme solution of Comparative Example C-1 and the enzyme solution of Comparative Example C-2, relative biodegradability of each specimen was evaluated in the same manner as above.

The entire process of the biodegradability evaluation was performed under 28 °C and normal pressure (760$\pm$20 torr).

(5) Result analysis

FIG. 9 is the biodegradability evaluation results in an industrial compost environment of PBAT compound 1 (PBAT C1), PBAT compound 2 (PBAT C2), and cellulose (manufacturer: Sigma-Aldrich, product number: 310697) measured according to ISO 14855 (Determination of the ultimate aerobic biodegradability of plastic materials under controlled composting conditions-Method by analysis of evolved carbon dioxide). Referring to FIG. 9, it can be confirmed that PBAT compound 2 has higher biodegradability than PBAT compound 1.

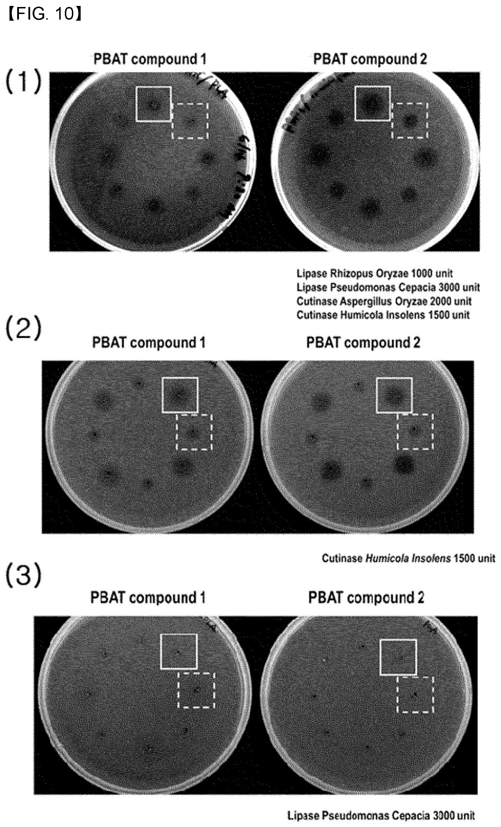

FIG. 10 is the biodegradability evaluation result of (4), and is a photo taken of the specimen 4 hours and 30 minutes after dropping the hydrolase. FIG. 10 was taken with a black screen added under the specimen, and the part that looks black is the clear zone. The results of Example C-1 are indicated by (1), the results of Comparative Example C-1 by (2), and the results of Comparative Example C-2 by (3).

Referring to (1) of FIG. 10, when the same time elapsed after dropping the enzyme solution of Example C-1, it can be confirmed that the clear zone (black looking part) of the specimen of PBAT compound 2, which has excellent biodegradability, appeared more clearly than the specimen of PBAT compound 1.

However, referring to (2) and (3) of FIG. 10, it can be confirmed that no significant difference between PBAT compound 1 and PBAT compound 2 can be observed with the enzyme solution of Comparative Example C-1 containing only cutinase and the enzyme solution of Comparative Example C-2 containing only lipase.

Accordingly, it can be confirmed from the above experimental results that relative biodegradability in a soil/compost environment of biodegradable resin can be simply and reliably predicted by the biodegradability evaluation method for biodegradable resin using the enzyme solution of the present disclosure.

**Example C-2: Biodegradability evaluation in a marine environment**

(1) Preparation of medium

A medium was prepared in the same manner as in (1) of Example C-1.

(2) Preparation of resin solution

1) Preparation of P3HP 1 solution

Poly-3-hydroxypropionic acid (P3HP, LG Chem) having a weight average molecular weight (Mw) of 120,000 g/mol disinfected with 70% ethanol was dissolved in 20 ml of chloroform at 1% by weight to prepare a P3HP 1 solution.

2) Preparation of P3HP 2 solution

P3HP (LG Chem) having a weight average molecular weight (Mw) of 70,000 g/mol disinfected with 70% ethanol was dissolved in 20 ml of chloroform at a concentration of 1% by weight to prepare a P3HP 2 solution.

(3) Preparation of enzyme solution

An enzyme solution (stock solution) was prepared by mixing 1 type of lipase and 1 type of cutinase below with 1 ml of 1X Phosphate-Buffered Saline (PBS). In addition, a 1/10 dilution of the stock solution was prepared.

Lipase *Pseudomonas Cepacia* 3000 unit
Cutinase *Humicola Insolens* 1500 unit

(4) Biodegradability evaluation

Using each resin solution of (2) and the enzyme solution of (3), biodegradability evaluation of P3HP 1 and P3HP 2 resins was performed in the same manner as in (4) of Example C-1.

(5) Result analysis

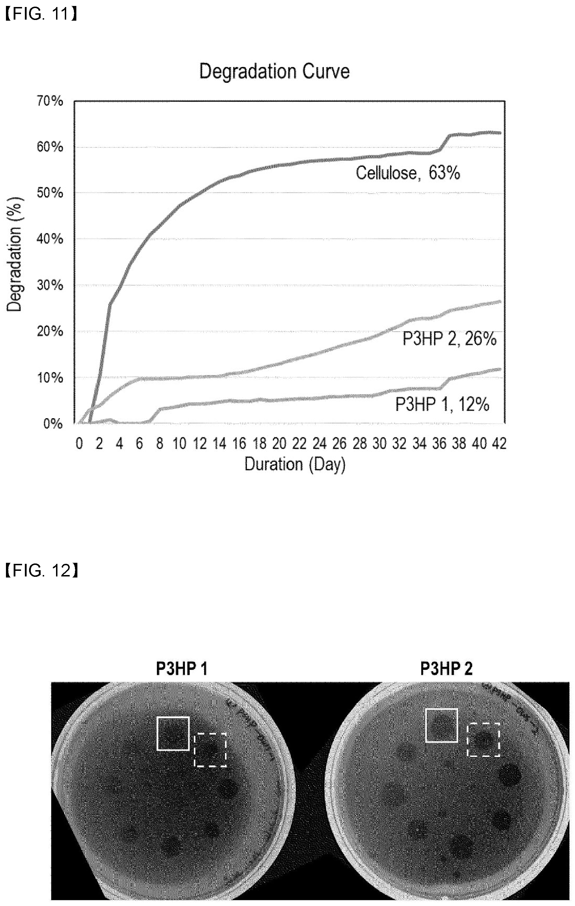

FIG. 11 is the biodegradability evaluation results in a marine environment of P3HP 1, P3HP 2, and cellulose (manufacturer: Sigma-Aldrich, product number: 310697) measured according to the ASTM D6691 standard. Referring to FIG. 11, it can be confirmed that P3HP 2 has higher biodegradability than P3HP 1.

FIG. 12 is the biodegradability evaluation result of (4), and is a photo taken of the specimen 18 hours after dropping the hydrolase. When the same time elapsed, it can be confirmed that the clear zone of the specimen of P3HP 2, which has excellent biodegradability, appeared more clearly than the specimen of P3HP 1.

Accordingly, it can be confirmed from the above experimental results that relative biodegradability in a marine environment of biodegradable resin can be simply and reliably predicted by the biodegradability evaluation method for biodegradable resin using the enzyme solution of the present disclosure.

**Example D-1: Biodegradability evaluation and image analysis**

(1) Preparation of medium

A circular 1% agar plate with a diameter of 90 mm was prepared by a method of preparing a Petri dish medium.

A solution was prepared by adding 10 g of agar (Manufacturer: BD Difco, product name: Bacto Agar, product number: 214010) and 1000 ml of distilled water to a 2000 ml Erlenmeyer flask. The solution was put into an autoclave and sterilized at a temperature of 121°C and a pressure of 0.1 MPa for 15 minutes. The sterilized solution was dispensed into sterile circular Petri dishes with a diameter of 90 mm in 10 ml portions to prepare a medium.

(2) Preparation of biodegradable resin solution

A resin solution was prepared by completely dissolving biodegradable resin in 20 ml of chloroform at a concentration of 1 wt%.

(3) Preparation of enzyme solution

An enzyme solution was prepared by mixing 2 types of lipase, 2 types of cutinase, and 1 type of alpha-amylase below with 1 ml of 1X Phosphate-Buffered Saline (PBS).

Lipase *Rhizopus Oryzae* 1000 unit
Lipase *Pseudomonas Cepacia* 3000 unit
Cutinase *Aspergillus Oryzae* 2000 unit
Cutinase *Humicola Insolens* 1500 unit
$\alpha$-amylase *Bacillus Licheniformis* 1500 unit

(4) Biodegradability evaluation and image acquisition

On the agar plate of (1), 1 to 3 ml of the resin solution prepared in (2) was evenly sprayed and dried at room temperature (25°C) for about 10 minutes so that the solvent was volatilized, thereby preparing a specimen having a thickness of 0.7 $\mu$m.

The agar plate was sealed and mounted on JuLI™ Stage (manufactured by NanoEntek), which is image measurement/analysis equipment. The enzyme solution prepared in (3) was dropped onto the biodegradable resin layer on the agar plate. Real-time images were acquired at certain time intervals while maintaining the image acquisition (shooting) conditions constant using JuLI™ Stage.

The measurement conditions are summarized in the table below.

[Table 3]

| Sample No. | Resin | Agar injection amount | Resin sprayin 9 amount | Enzyme solution concentration (ratio) | Interval |
|---|---|---|---|---|---|
| 1 | PBAT C1 | 0.6ml | 4ml | 1 | 22min |
| 2-1 | PBAT C2 | 0.7ml | 4ml | 1 | 39min |
| 2-2 | PBAT C1 | 0.7ml | 4ml | 1 | 39min |
| 3-1 | PBSA C1 | 0.7ml | 2ml | 1/10 dilution | 11min |
| 3-2 | PBSA C2 | 0.7ml | 2ml | 1/10 dilution | 11min |
| 3-3 | PBSA C3 | 0.7ml | 2ml | 1/10 dilution | 11min |
| 3-4 | PBSA C4 | 0.7ml | 2ml | 1/10 dilution | 11min |

(5) Image analysis

By analyzing the images obtained above, the brightness of the image immediately after dropping the enzyme and the brightness of the image taken after each elapsed time were confirmed, and the average value of the brightness difference was plotted against elapsed time. The curve obtained by plotting was fitted to a sigmoid function, and the elapsed time at which the second derivative of the function was at its maximum was confirmed.

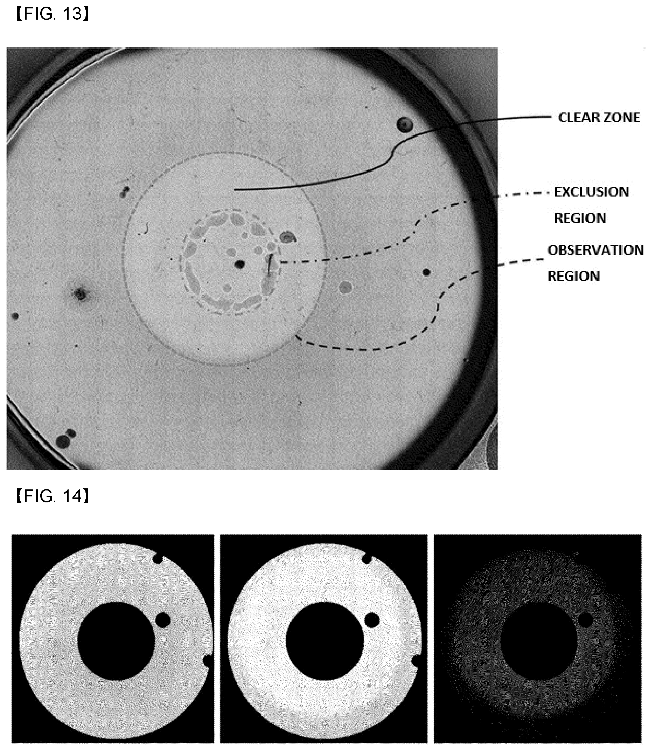

FIG. 13 is an image showing the degree of biodegradation of biodegradable resin obtained according to an embodiment of the present disclosure.

Referring to FIG. 13, in the region where the biodegradable resin is located, an "observation region" where the white biodegradable resin is hydrolyzed in the middle of the corresponding region and appears in a lighter color than the periphery can be identified, and in the "observation region", an "exclusion region" where other residues generated during the process of dropping the enzyme on the resin, rather than those formed by hydrolysis by the reaction of the enzyme and the resin, form a dark background color through interaction with the resin can be identified.

That is, as the hydrolysis progresses, the brightness of the biodegradable resin region becomes brighter, and then, if the hydrolysis exceeds a certain point, an "exclusion region" occurs which actually becomes darker. Therefore, for accurate evaluation, it is necessary to exclude the exclusion region from evaluation, and in other cases where the brightness is exceptionally different from the periphery, it is also necessary to exclude it from evaluation.

FIG. 14 is an image showing the degree of biodegradation of biodegradable resin obtained according to an embodiment of the present disclosure.

More specifically, in FIG. 14, the left drawing is an image taken immediately after dropping the hydrolase, post-processed to exclude the exclusion region (black circle) from the observation region. In FIG. 14, the middle drawing is an image taken after unit time has elapsed after dropping the hydrolase, also post-processed to exclude the exclusion region from the observation region, and it can be confirmed that the brightness of the observation region is somewhat brighter than the left drawing. The right drawing in FIG. 14 shows the calculated brightness difference between the left drawing and the middle drawing.

By the treatment as described above, the existence of a brightened region and the brightness difference can be confirmed.

The average value of the brightness differences obtained above was obtained, plotted against the elapsed time when the image was taken, and fitted in the form of a Sigmoid function.

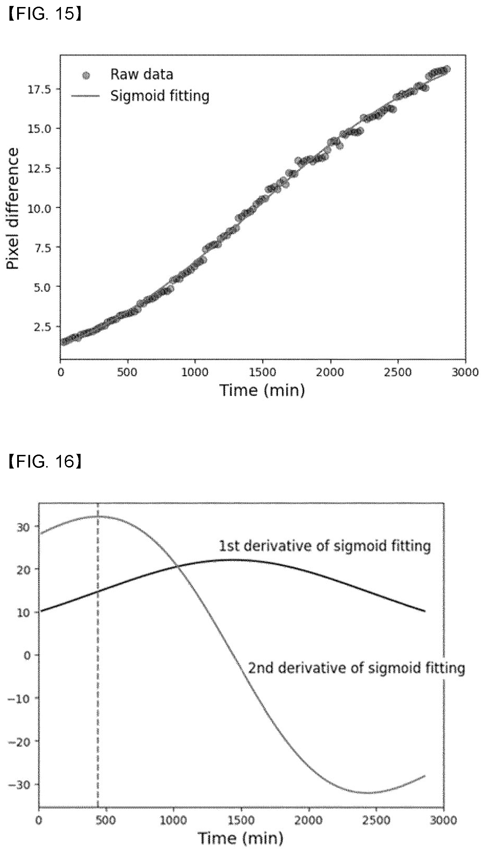

FIG. 15 is a graph showing the image brightness difference over time and the fitted function obtained according to an embodiment of the present disclosure.

The form of the Sigmoid function used for fitting in FIG. 15 is as follows. At this time, t is time in minutes (min), a, b, c, d are fitting constants obtained through fitting, and e is a natural constant (Euler's Number).

$$f(t) = \frac{a}{(1 + e^{-bt+c})} + d$$

And, the first derivative function and second derivative function of this function are as follows, respectively.

$$f'(t) = \frac{abe^{-bt+c}}{(1 + e^{-bt+c})^2},$$

$$f''(t) = \frac{ab^2e^{-bt+c}}{(1 + e^{-bt+c})^3}(e^{-bt+c} - 1)$$

FIG. 16 is a graph showing a first derivative function and a second derivative function obtained according to an embodiment of the present disclosure.

Referring to FIG. 16, in the second derivative function, a maximum value appears in the measurement range. This point can be interpreted as a point in time when the brightness change of the image proceeds fastest (a point in time when the change acceleration of the biodegradable resin has the maximum value), and this point in time can be designated as a point in time when hydrolysis of the resin occurs and a clear zone begins to occur.

After obtaining the second derivative function and confirming the elapsed time t at which the second derivative is the maximum value, they were summarized in the table below.

[Table 4]

| Sample No. | Time(min) |
|---|---|
| 1 | 440 |
| 2-1 | 600 |

(continued)

| Sample No. | Time(min) |
|------------|-----------|
| 2-2 | 1040 |
| 3-1 | 594 |
| 3-2 | 319 |
| 3-3 | 176 |
| 3-4 | 143 |

Referring to Table 4, the time when the clear zone begins to occur for each experiment can be confirmed. If this is compared under the same experimental conditions, i.e., by interval, in the case of Sample No. 2-1 (PBAT C2), it can be seen that the clear zone begins to occur earlier than in Sample No. 2-2 (PBAT C1). If this is calculated as a reciprocal, it can be confirmed that No. 2-1 (PBAT C2) has about 70% better biodegradability than No. 2-2 (PBAT C1). In the case of PBSA, it can be seen that the time when the clear zone begins to occur gradually becomes faster from No. 3-1 to No. 3-4, and regarding the biodegradability of PBSA, No. 3-4 (PBSA C4) is the best, followed by No. 3-3 (PBSA C3), No. 3-2 (PBSA C2), and No. 3-1 (PBSA C1).

FIG. 9 shows the biodegradability evaluation results in a soil environment of PBAT compound 1 (PBAT C1), PBAT compound 2 (PBAT C2), and cellulose (manufacturer: Sigma-Aldrich, product number: 310697) measured according to ISO 14855 (Determination of the ultimate aerobic biodegradability of plastic materials under controlled composting conditions-Method by analysis of evolved carbon dioxide). Referring to FIG. 9, it can be confirmed that PBAT compound 2 (PBAT C2) has higher biodegradability than PBAT compound 1 (PBAT C1).

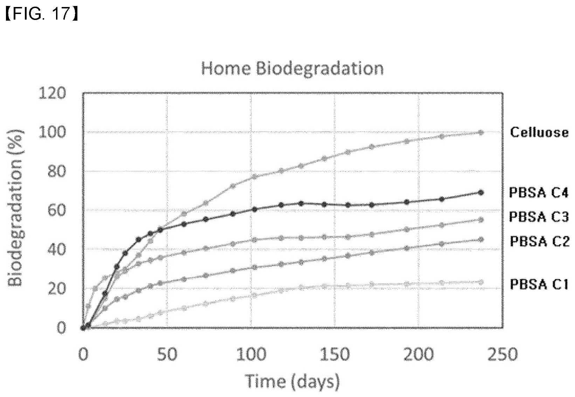

FIG. 17 shows the biodegradability evaluation results in home compost of PBSA compound 1 (PBSA C1), PBSA compound 2 (PBSA C2), PBSA compound 3 (PBSA C3), PBSA compound 4 (PBSA C4), and cellulose (manufacturer: Sigma-Aldrich, product number: 310697) measured in accordance with the ISO 14855-1 standard. Referring to FIG. 17, it can be confirmed that they have higher biodegradability in the order of PBSA C4 > PBSA C3 > PBSA C2 > PBSA C1.

From the above results, it can be seen that the results according to the image analysis of the present disclosure are consistent with the biodegradability evaluation results of biodegradable resin according to the ISO standard.

**Claims**

1.  An enzyme solution comprising:

    a hydrolase comprising lipase and cutinase; and
    phosphate-buffered saline.

2.  The enzyme solution of claim 1,
    wherein an enzyme unit ratio of lipase:cutinase is 0.5:1 to 4:1.

3.  The enzyme solution of claim 1 or 2,
    wherein a total amount of hydrolase in 1 ml of the enzyme solution is 400 unit to 80,000 unit.

4.  The enzyme solution of any one of claims 1 to 3,
    wherein the lipase is a lipase of one or more microorganisms selected from the group consisting of *Pseudomonas Cepacia, Rhizopus Oryzae, Aspergillus niger,* and *Aspergillus Oryzae.*

5.  The enzyme solution of any one of claims 1 to 4,
    wherein the cutinase is a cutinase of one or more microorganisms selected from the group consisting of *Humicola Insolens, Aspergillus Oryzae, Fusarium solani,* and *Pseudomonas putida.*

6.  The enzyme solution of any one of claims 1 to 5,

    wherein the lipase includes lipase of *Pseudomonas Cepacia,* and
    the cutinase includes cutinase of *Humicola Insolens.*

7.  The enzyme solution of any one of claims 1 to 6,

comprising lipase of *Pseudomonas Cepacia* and lipase of *Rhizopus Oryzae* as the lipase, and comprising cutinase of *Humicola Insolens* and cutinase of *Aspergillus Oryzae* as the cutinase.

8.  The enzyme solution of any one of claims 1 to 7,

comprising lipase of *Pseudomonas Cepacia* as the lipase, and comprising cutinase of *Humicola Insolens* and cutinase of *Aspergillus Oryzae* as the cutinase.

9.  The enzyme solution of any one of claims 1 to 8, further comprising one or more enzymes selected from the group consisting of $\alpha$-amylase, cellulase, proteinase K, protease, acetyl-CoA carboxylase, and alcalase.

10. A biodegradability evaluation method for biodegradable resin comprising:

i) contacting an enzyme solution comprising a hydrolase comprising lipase and cutinase, and phosphate-buffered saline with a biodegradable resin; and
ii) observing a degradation state of the biodegradable resin over time.

11. The biodegradability evaluation method for biodegradable resin of claim 10, wherein i) is performed by dropping the enzyme solution onto a resin specimen comprising biodegradable resin.

12. The biodegradability evaluation method for biodegradable resin of claim 10, wherein i) is performed by immersing a resin specimen comprising biodegradable resin in the enzyme solution.

13. The biodegradability evaluation method for biodegradable resin of claim 10,

wherein i) is performed by spraying a biodegradable resin solution onto a medium to prepare a specimen, and then dropping the enzyme solution onto the specimen, and
ii) is performed by observing a change in a clear zone formed on the specimen over time.

14. The biodegradability evaluation method for biodegradable resin of any one of claims 10 to 13, wherein ii) includes:

a) obtaining an image of the biodegradable resin according to elapsed unit time; and
b) evaluating biodegradability through a change in the image of the resin specimen over time.

15. The biodegradability evaluation method for biodegradable resin of claim 14, wherein b) includes:

b-1) for each image of the resin specimen obtained according to elapsed unit time, obtaining a brightness difference with an image immediately after dropping a hydrolase;
b-2) representing image brightness difference values as a function of elapsed unit time; and
b-3) evaluating biodegradability through analysis of the function.

16. The biodegradability evaluation method for biodegradable resin of claim 15, wherein b-3) evaluates the biodegradability of the biodegradable resin by confirming a time when a second derivative of the function is a maximum value.

【FIG. 1】

【FIG. 2】

【FIG. 3】

10 DPI (day post inoculation)

PBS

PBSA

8.9 mm²      21.2 mm²

【FIG. 4】

Degradation Curve %

【FIG. 5】

EP 4 779 304 A1

【FIG. 6】

【FIG. 7】

23

【FIG. 8】

【FIG. 9】

【FIG. 10】

(1) PBAT compound 1  PBAT compound 2

Lipase Rhizopus Oryzae 1000 unit
Lipase Pseudomonas Cepacia 3000 unit
Cutinase Aspergillus Oryzae 2000 unit
Cutinase Humicola Insolens 1500 unit

(2) PBAT compound 1  PBAT compound 2

Cutinase *Humicola Insolens* 1500 unit

(3) PBAT compound 1  PBAT compound 2

Lipase Pseudomonas Cepacia 3000 unit

【FIG. 11】

Degradation Curve

【FIG. 12】

【FIG. 13】

CLEAR ZONE

EXCLUSION
REGION

OBSERVATION
REGION

【FIG. 14】

【FIG. 15】

【FIG. 16】

【FIG. 17】

**INTERNATIONAL SEARCH REPORT**

International application No.

**PCT/KR2025/013565**

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☑ Claims Nos.: **4-9**
   because they are dependent claims and are not drafted in accordance with the second and third sentences of Rule 6.4(a).

Form PCT/ISA/210 (continuation of first sheet) (July 2022)

## INTERNATIONAL SEARCH REPORT

| International application No. |
|---|
| **PCT/KR2025/013565** |

| | |
|---|---|
| **A.** | **CLASSIFICATION OF SUBJECT MATTER** |
| | **G01N 33/44**(2006.01)i; **C12Q 1/02**(2006.01)i; **C12Q 1/34**(2006.01)i |

According to International Patent Classification (IPC) or to both national classification and IPC

| | |
|---|---|
| **B.** | **FIELDS SEARCHED** |

Minimum documentation searched (classification system followed by classification symbols)

G01N 33/44(2006.01); C08J 11/10(2006.01); C08J 11/16(2006.01); C08J 11/18(2006.01); C12N 9/18(2006.01); C12N 9/20(2006.01); C12Q 1/02(2006.01); G06F 19/00(2011.01); G06K 9/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (MOIP internal) & keywords: 라이페이즈(lipase), 큐티네이즈(cutinase), 가수분해(hydrolysis), 인산염 완충 식염수(phosphate-buffered saline), 효소(enzyme), 생분해성(biodegradable), 클리어존(clear zone), 밝기(brightness), 함수(function)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | US 2024-0067793 A1 (SOCIETE DES PRODUITS NESTLE S.A.) 29 February 2024 (2024-02-29) claims 1, 5; paragraphs [0037], [0071]-[0075] | 1-3,10-12 |
| Y | | 13-14 |
| A | | 15-16 |
| Y | US 2018-0051264 A1 (CARBIOS et al.) 22 February 2018 (2018-02-22) paragraphs [0167], [0179], [0186] | 13-14 |
| A | JP 2016-023194 A (NATIONAL INSTITUTE FOR AGRO-ENVIRONMENTAL SCIENCE) 08 February 2016 (2016-02-08) paragraphs [0030], [0034]-[0035] | 1-3,10-16 |

☑ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **28 November 2025** | **30 November 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**TRANSLATION**

**INTERNATIONAL SEARCH REPORT**

| International application No. |
| --- |
| **PCT/KR2025/013565** |

### C. DOCUMENTS CONSIDERED TO BE RELEVANT

| Category* | Citation of document, with indication, where appropriate, of the relevant passages | Relevant to claim No. |
| --- | --- | --- |
| A | KR 10-1439918 B1 (INDUSTRY-ACADEMIC COOPERATION FOUNDATION, YONSEI UNIVERSITY) 15 September 2014 (2014-09-15)<br>claims 1-5; figures 1-4 | 1-3,10-16 |
| A | US 2009-0310839 A1 (KATZENELSON, O. Y. et al.) 17 December 2009 (2009-12-17)<br>claim 1; figure 1 | 1-3,10-16 |

Form PCT/ISA/210 (second sheet) (July 2022)

**INTERNATIONAL SEARCH REPORT**
Information on patent family members

| International application No. |
|---|
| **PCT/KR2025/013565** |

| Patent document cited in search report | | | Publication date (day/month/year) | Patent family member(s) | | | Publication date (day/month/year) |
|---|---|---|---|---|---|---|---|
| US | 2024-0067793 | A1 | 29 February 2024 | CN | 116529317 | A | 01 August 2023 |
| | | | | EP | 4267666 | A1 | 01 November 2023 |
| | | | | JP | 2024-500072 | A | 04 January 2024 |
| | | | | MX | 2023006358 | A | 13 June 2023 |
| | | | | WO | 2022-135988 | A1 | 30 June 2022 |
| US | 2018-0051264 | A1 | 22 February 2018 | CN | 107532153 | A | 02 January 2018 |
| | | | | EP | 3268469 | A1 | 17 January 2018 |
| | | | | US | 10508269 | B2 | 17 December 2019 |
| | | | | WO | 2016-146540 | A1 | 22 September 2016 |
| JP | 2016-023194 | A | 08 February 2016 | JP | 6338183 | B2 | 06 June 2018 |
| KR | 10-1439918 | B1 | 15 September 2014 | US | 2015-0087012 | A1 | 26 March 2015 |
| US | 2009-0310839 | A1 | 17 December 2009 | WO | 2009-152389 | A2 | 17 December 2009 |
| | | | | WO | 2009-152389 | A3 | 22 April 2010 |

Form PCT/ISA/210 (patent family annex) (July 2022)

**REFERENCES CITED IN THE DESCRIPTION**

**Patent documents cited in the description**

- KR 1020240119312
- KR 1020240119313
- KR 1020240119314
- KR 1020240183657